Europäisches Patentamt

European Patent Office

Office européen des brevets

(11) Publication number:

**0 347 915**
**A2**

# EUROPEAN PATENT APPLICATION

(21) Application number: 89111394.6

(22) Date of filing: 22.06.89

(51) Int. Cl.⁴: **A61K 37/22 , C12N 15/00**

(30) Priority: 23.06.88 US 211546

(43) Date of publication of application:
**27.12.89 Bulletin 89/52**

(84) Designated Contracting States:
**AT BE CH DE ES FR GB GR IT LI LU NL SE**

(71) Applicant: THE ROCKEFELLER UNIVERSITY
1230 York Avenue
New York New York 10021-6399(US)

(72) Inventor: Breslow Jan L.
7 Horseguard Lane-Scarsdale
New York, New York 10583(US)
Inventor: Todd Leff
500 East 63rd Street
New York, New York 10021(US)

(74) Representative: Jorio, Paolo et al
STUDIO TORTA Società Semplice Via Viotti 9
I-10121 Torino(IT)

(54) **Method and agents for inhibiting low density lipoprotein synthesis.**

(57) The present invention in a first aspect defines a method of inhibiting the synthesis of low density lipoproteins (LDL) which comprises administration of a substance which blocks Apo B gene transcription by interfering with the interaction of transacting proteins with the cis-acting DNA regulatory regions of the Apo B gene between the -128 and -60 sequence elements. The various inhibitory substances can be administered to patients whose medical condition would benefit from a lowering of their plasma LDL levels. The invention also includes agents and pharmaceutical compositions and their preparation, which agents and compositions are suitable for the practice of the inventive method. Diagnostic and assay procedures are also included.

EP 0 347 915 A2

## METHOD AND AGENTS FOR INHIBITING LOW DENSITY LIPOPROTEIN SYNTHESIS

This invention was made with partial assistance from grants from the National Institutes of Health, the American Heart Association and the Pew Trust.

### RELATED PUBLICATIONS

The Applicant is a co-author of the following article directed to the subject matter of the present invention: "CELL TYPE SPECIFIC EXPRESSION OF THE HUMAN APO B GENE IS CONTROLLED BY TWO CIS-ACTING REGULATORY REGIONS" (in press), Hriday K. Das, Todd Leff and Jan L. Breslow.

### BACKGROUND OF THE INVENTION

The present invention is directed generally to the study of the apolipoprotein apo B, and particularly to the regulation of its activity and the consequent effect on the level of hepatic low density lipoproteins (LDL) in a mammal, through the modulation of the transcription of the apo B gene in the liver.

Apolipoproteins are important constituents of lipoprotein particles and participate in lipoprotein synthesis, secretion, processing and catabolism. One of these, apo B exists in two form, B-100 and B-48. In humans, B-100 is synthesized in the liver and is a constituent of very low density lipoproteins (VLDL), intermediate density lipoproteins (IDL) and low density lipoproteins (LDL). B-100 is the major protein portion of LDL and is the ligand for the LDL receptor. B-48 is synthesized in the small intestine and is a constituent of chylomicrons and their remnants. Recently, the gene for apo B has been cloned, found to be single copy and mapped to human chromosome 2pter-2p24. This gene is 43 kb in length, comprised of 29 exons and 28 introns, and codes for a 27 amino acid signal peptide and a mature protein of 4536 amino acids. B-100 and B-48 are products of one gene and have the same amino terminal amino acid sequences. It appears that in the intestine there is tissue specific processing of apo B mRNA, which introduces a stop sequence at codon 2136. This not present in liver apo B mRNA or in the genomic DNA from liver or intestine.

Plasma levels of LDL cholesterol and apo B (B-100) correlate directly with coronary heart disease incidence. Elevated apo B levels are seen in two autosomal dominant genetic disorders associated with premature atherosclerosis, familial hypercholesterolemia and familial combined hyperlipoproteinemia. The former is characterized by a decreased apo B plasma fractional catabolic rate and due to a deficiency of LDL receptors. The latter is associated with increased apo B synthesis of unknown cause. Clinical disorders associated with diminished apo B levels called abetalipoproteinemia and heterozygous and homozygous hypobetalipoproteinemia also exist. The cause of abetalipoproteinemia is not known, but hpobetalipoproteinemia was recently shown to be due to defects in the apo B gene, Leppert et al., J. Clin. Invest., in press. In this study, the hypobetalipoproteinemia phenotype co-segregated in a family with one particular apo B gene haplotype established by restriction fragment length polymorphism analysis. The LOD score was greater than 7. In another study, hypobetalipoproteinemia co-segregated with a mutant form of the apo B protein. These studies indicate that diminished apo B levels can be caused by regulatory and/or structural defects in the apo B gene.

In view of the clinical consequences of apo B presence and activity, a need exists to further elucidate the mechanism of its action and to develop diagnostic and therapeutic protocols based thereon.

### SUMMARY OF THE INVENTION

In one aspect, the present invention relates to a method and agents for inhibiting low density lipoprotein (LDL) synthesis by interfering with the transcription of the apo B gene in the liver. More particularly, this aspect of the invention comprises a method for blocking apo B gene transcription in the liver by administering an inhibitory amount of an agent comprising a substance that can interfere with the interaction of trans-acting proteins and the cis-acting DNA regulatory regions between the -128 to -60 sequence elements of the apo B gene, which control apo B gene transcription.

The particular inhibitory substances or agents can be identified by examination of their effects upon the interaction of transacting proteins and the cis-acting DNA regulatory regions between the -128 to -60 sequence elements of the apo B gene, and appropriate investigative procedures such as defined herein comprise a further aspect of the invention relating to a screening assay.

Also, diagnostic utility resides in the measurement of apo B transcription and in the consequent assessment of susceptability to the genetic or pathological disorders attributable to excessive plasma LDL, all in accordance with a further aspect of the invention.

It is accordingly a principal object of the present invention to provide a method and agents for inhibiting low density lipoprotein (LDL) synthesis.

It is also an object of the present invention to provide a method of treating individuals whose medical condition would benefit from a lowering of their plasma LDL levels.

It is still a further object of the present invention to provide a method of identifying the agents which would function to lower plasma LDL levels by their ability to interfere with the interaction of trans-acting proteins and the cis-acting DNA regulatory regions of the apo B gene.

It is a yet further object of the present invention to provide methods for diagnosing the onset of conditions due to abnormalities in plasma LDL levels by measuring the presence and transcriptional activity of the apo B gene.

## DESCRIPTION OF THE DRAWINGS

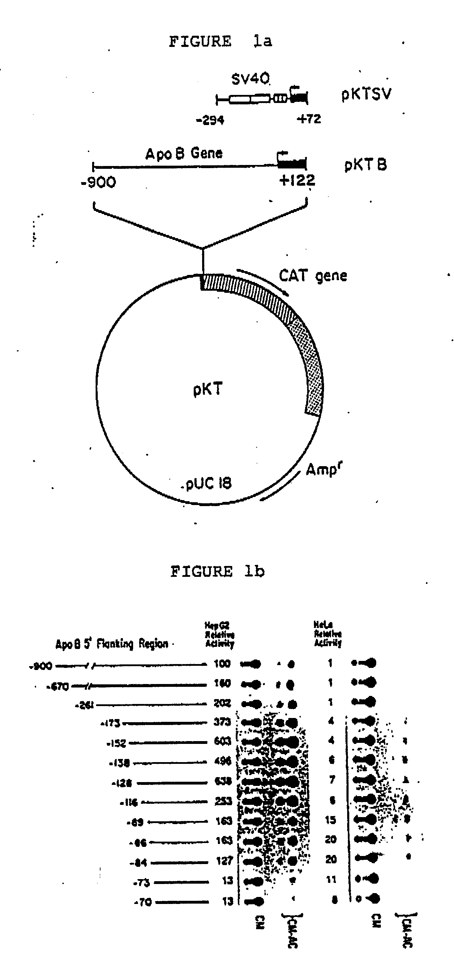

FIGURE 1A is a diagram showing the structure of plasmid vector pKT and derivatives (see Example 1 for details of construction). pKT contains the entire CAT gene coding sequence (hatched region) and SV40 splice site and polyadenylation signal (stippled region) inserted into pUC18. pKTB contains human apo B gene sequences from -900 to +122 (relative to the start site of transcription) ligated into the polyliner of pKT (solid box on pKT map). pKTSV contains SV40 early promoter sequences, including the two 72 bp and three 21 bp repeats (open boxes), inserted into the pKT polylinker. Solid boxes and arrows in pKTB and pKTSV represent exonic sequences and transcription start sites, respectively.

FIGURE 1B is a representative CAT assay showing the stimulation of CAT gene expression of apo B 5′ flanking DNA sequences. A series of plasmids containing deletions in the 5′ flanking DNA were constructed from pKTB and their activity in HepG2 and HeLa cells determined as described in Experimental Procedures. Activities are expressed relative to that achieved with the -900 construction in HepG2 cells and represent the average of at least three separate transfections. A representative CAT assay is shown with unreacted chloramphenicol (CM) and acetylated products (CM-Ac) indicated. All experiments were normalized for transfection efficiency using $\beta$-galactosidase activity as described in experimental procedures.

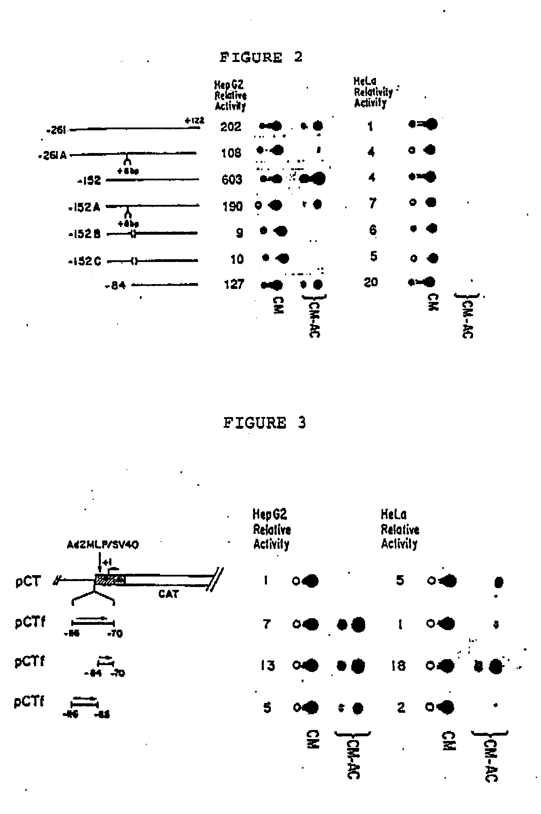

FIGURE 2 is an analysis of the transcriptional activity of the apo B proximal positive and negative element. Mutations were produced by inserting an 8 bp XhoI linker into the Sma I site at -85 (-261A and -152A) or deletion of sequences from -84 to -72 (152B) and from -84 to -63 (152C). Transcriptional activities are expressed as a percent activity of a pKTB/-900 construction run in the same series. The relative activities represent the averages from three separate experiments. CAT activities were normalized for transfection efficiency using the $\beta$-galactosidase activity (see Examples). Unreacted chloramphenicol (CM) and acetylated products (CM-Ac) are indicated.

Figure 3 is an analysis of the apo B proximal positive and negative elements in a heterologous promoter. Apo B DNA fragments containing sequences from -116 to -70, -84 to -70, and -116 to -85 were inserted into the vector pCT (described in Examples section). pCT contains the adenovirus 2 major late promoter (hatched box) and untranslated leader sequences from V (stippled) located upstream of the CAT gene (open box). Apo B sequences were inserted 55 bp upstream of the major late transcription start site (designated +1) in forward orientation. Transcriptional activities of these constructions represent the average of three experiments and are expressed relative to the basal level of pCT activity of the HepG2 series and relative to the pCTf 116/70 construction for the HeLA series. CAT activity was normalized with $\beta$-galactosidase activity for each transfection experiment (see Examples section).

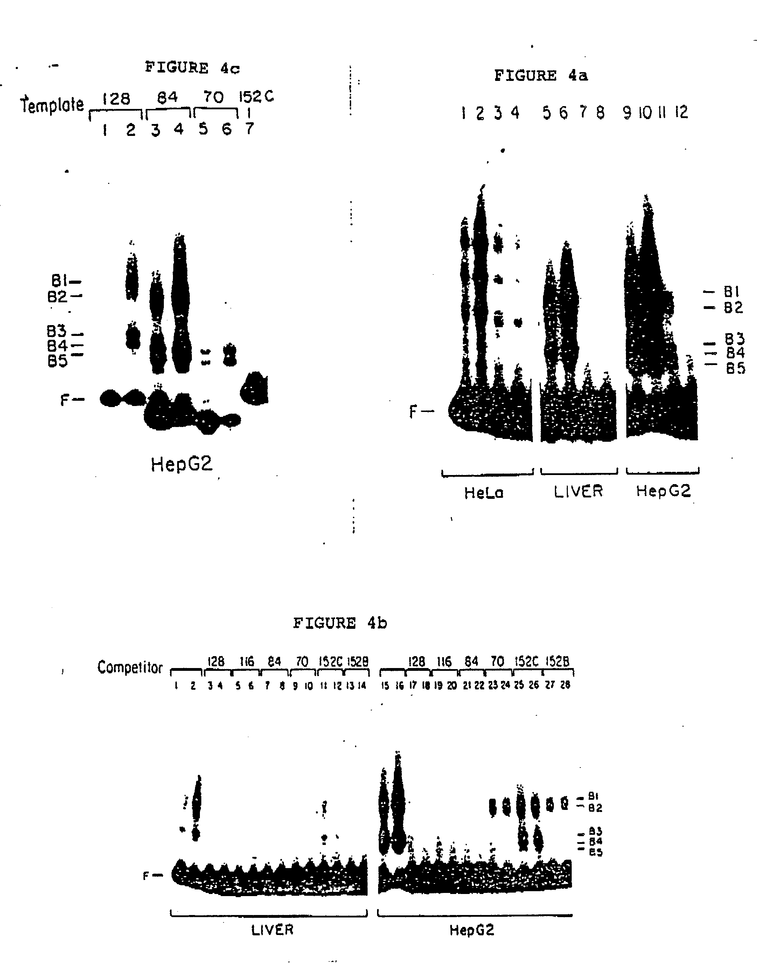

FIGURE 4A is an electrophoretic mobility shift assay showing the incubation of nuclear extracts prepared from HeLa and HepG2 cells and from mouse liver with a radio-labeled DNA fragment containing apo B sequences from -128 to +122 (1.2 ng DNA = 15,000 cpm). Lanes 1, 3, 4 contain 3.5 $\mu$g and lane 2 contains 7 $\mu$g of HeLa protein. Lanes 5, 7, 8 contain 1 $\mu$g and lane 6 contains 2 $\mu$g of liver protein. Lanes 9, 11, 12 contain 2.1 $\mu$g and lane 10 contains 4.2 $\mu$g of HepG2 protein. Fifty-fold molar excess of an unlabelled DNA competitor fragment (-128 to +122) was used for lanes 3, 7 and 11. Lanes 4, 8 and 12 contain 100-fold molar excess of unlabeled competitor. "F" denotes the free (unbound) fragment, and B1-B5 indicate the position of bands representing protein-DNA complexes.

FIGURE 4B is an assay showing the competition of specific DNA- protein complexes by various DNA fragments of the apo B promoter. A radio-labeled DNA fragment containing apo B sequences from -128 to +122 was incubated with mouse liver and HepG2 cell nuclear extracts and protein DNA complexes were analyzed as indicated in Example 3. Different apo B promoter fragments were used as specific competitors (see Example 3). Odd-numbered lanes contain a 50-fold molar excess of unlabeled competitors, while even-numbered lanes contain a 100-fold molar excess of specific competitors as indicated. Lanes 1, 3-14 contain 1 μg and lane 2 contains 2 μg of liver protein. Lanes 15, 17-28 contain 2.1 μg and lane 16 contains 4.2 μg of HepG2 protein.

FIGURE 4C is an assay showing the binding of protein factors in HepG2 cell nuclear extracts to radio-labeled apo B promoter fragments 128 (-128 to +122), 84 (-84 to +122), 70 (-70 to +122) and 152C (see Figure 2) (1.1 ng). Lanes 1, 3, 5 contain 2.1 μg and lanes 2, 4, 6 and 7 contain 4.2 μg of HepG2 protein. "F" denotes the free template and B1-B5 indicate the positions of DNA protein complexes.

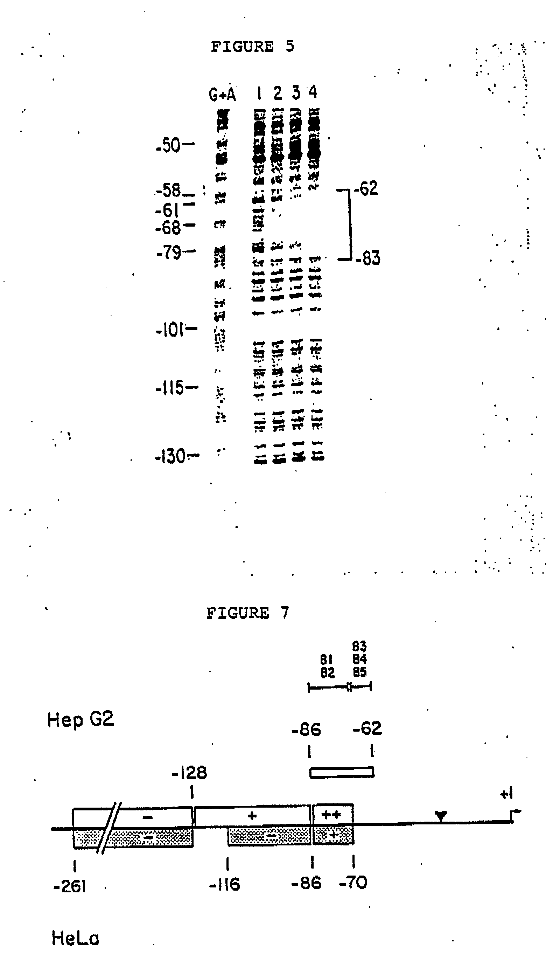

FIGURE 5 is a DNAse I footprint analysis of HepG2 proteins binding to the apo B promoter. A fragment of the apo B promoter (10 ng) extending from -261 to +122 was radio-labeled at the 5′ end of the coding strand and was incubated in the presence and absence of HepG2 nuclear protein and then treated with DNAse I (see Examples). A sequencing reaction of (G+A) of the same labeled fragment was run as a marker. The bracket indicates the position of sequence motifs protected from DNAse I cleavage by bound protein factor(s). Numbering begins at the transcription initiation site. Lane 1 contains no HepG2 extract and 60 μg of BSA, Lanes 2, 3 and 4 contain 12 μg, 24 μg and 48 μg of HepG2 nuclear extract, respectively.

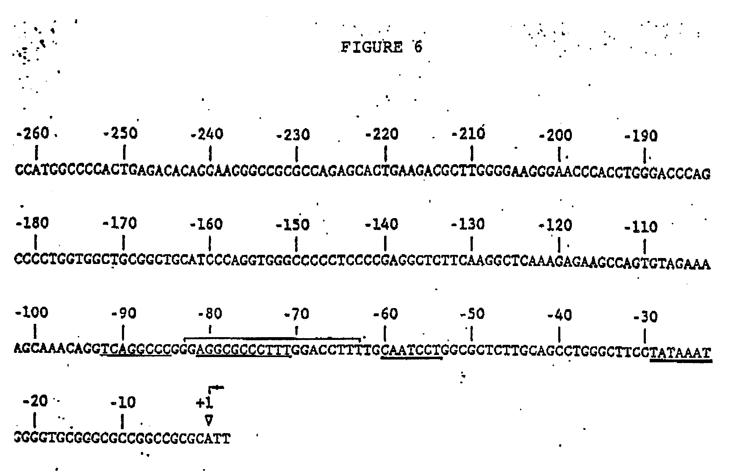

FIGURE 6 is the nucleotide sequence of human apo B 5′ flanking region. The sequence presented for human apo B confirms that which was previously reported by Blackhart et al., J. Biol. Chem., 261, 15364-15367 (1986). Numbering is with respect to start site of transcription (+1). "TATA" box and "CAAT" box are indicated by double underlines. Sequences protected in the DNAse I footprint experiment are indicated by solid overlie. Sequence motifs referred to in the specification and in Table 1 (-116 to -86 and -86 to -70) are indicated by single underlines.

FIGURE 7 is a schematic diagram showing the human apo B regulatory regions active in HepG2 and HeLa cells. Open boxes delineate cis-acting sequences active in HepG2 cells; stippled boxes represent elements active in HeLa cells. The + and -symbols indicate positive and negative cis-acting elements, respectively and their relative strength. Binding sites for (B1, B2) and (B3, B4, B5) bands identified by the gel mobility shift assay are marked. The sequences identified by the footprinting assay as hepatic protein binding sites are indicated by the open rectangle.

## DETAILED DESCRIPTION OF THE INVENTION

This invention relates to a method of inhibiting the synthesis of low density lipoproteins (LDL). It further includes agents to be utilized in the claimed method and pharmaceutical compositions prepared therefrom, as well as methods for the identification of such agents or inhibitory substances comparable to a screening assay, and diagnostic methods related to the measurement of the transcriptional activity of the apo B gene.

More particularly, the present invention in one embodiment defines a new method for lowing low density lipoprotein (LDL) cholesterol levels in the blood by interfering with the transcription of the apo B gene in the liver. This gene codes for the major LDL protein. Liver apo B gene transcription is determined by a specific cis-acting DNA region and proteins that interact with this region. By specifically interfering with this interaction and decreasing apo B gene transcription, LDL cholesterol will be profoundly depressed, thus causing therapeutic benefit to the patient being so treated. The human apolipoprotein B (apo B) gene codes for two related proteins, apo B100 and apo B48. Apo B100 is synthesized in the liver, is the major protein constituent of low density lipoprotein (LDL), and serves as the ligand for the LDL receptor.

The method of the present invention effects the inhibition of the synthesis of low density lipoproteins (LDL) by modifying the ability of the apo B gene to produce LDL. By administering an inhibitory amount of the agent of the present invention, comprising a substance which interferes with the transcription of the apo B gene, the apo B gene is prevented from carrying out its direction of the synthesis of low density lipoproteins (LDL). Accordingly, the present therapeutic method would possibly be applicable to conditions resulting from excessive quantities of plasma LDL, such as premature atherosclerosis, familial hypercholesterolemia and familial combined hyperlipoproteinemia, as well as conditions resulting from diminished apo B levels, such as hypobetalipoproteinemia.

The substances which interfere with the binding of trans-acting proteins to the cis-acting DN regulatory region consisting of the -128 to -60 sequence elements of the apo B gene, can be utilized to interfere with the ability of the apo B gene to carry out the synthesis of low density lipoproteins.

An effective inhibitory amount of the agent or interfering substance can be determined by experimentation and will, of course, vary, depending upon the patient's age, weight and the particular medical condition for which he or she is being treated.

The substances which interfere with the binding of the transacting proteins to the cis-acting DNA regulatory region consisting of the -128 to -60 sequence elements of the apo B gene can be identified by an assay that evaluates the effect of the substance upon this binding. The trans-acting proteins to be utilized in the assay are prepared according to the methods of Dignam, et al., Nucl. Acids Res., 11, 1475-1489 (1983) or Gorski, et al., Cell. 47 767-776 (1986). The appropriate DNA fragment, typically radiolabeled, is then added, as well as the potentially interfering substance. By measuring the amount of "bound" protein, the interfering ability of a substance can be determined. This measurement can be carried out by any of several analytical methods, but electrophoresis, typically on polyacrylamide gels, is a preferred method of measurement. After a substance is identified as an interfering substance, its inhibitory concentration and appropriate dosage can be quantified by further experimentation at varying levels of concentration.

Examination of the transcriptional regulation of apo B gene expression by linking the apo B gene regulatory region from -900 to +122 (with respect to the apo B start site of transcription) to the CAT reporter gene reveals that when this construction was transfected into hepatoma (HepG2) and epithelial carcinoma (HeLa) cells, expression of the CAT gene was observed only in HepG2 cells. Thus, the cis-acting DNA sequence elements controlling this cell-type specific expression of the apo B gene reside in this regulatory region and interact with the protein transcription factors that determine tissue specific expression.

A 5' deletional analysis of the cis-acting regulatory region of the Apo B gene reveals the presence of positive and negative regulatory regions. The most distal of these regions, located from -261 to -128 (with respect to the start-site of transcription) was found to have a roughly equivalent negative activity in both HepG2 and HeLa cells. However, sequences located from -128 to -86 showed a positive activity in HepG2 cells and a negative activity in HeLa cells. Finally, a sequence element located between positions -86 and -70 was found to have a very strong positive effect in HepG2 cells and only a mild positive effect in HeLa cell. These two proximal regions located between -128 and -70 thus act together to determine the cell-type specific expression of the apo B gene in HepG2 and HeLa cells.

Using a gel mobility shift assay and the DNAse I footprinting technique, it can be demonstrated that DNA binding proteins from HepG2 and mouse liver nuclear extracts interact with the crucial positive region located between -86 and -70. This region was also found to contain sequences elements similar to sequences found in the promoters of other apolipoprotein genes, as well as other genes that are expressed in the liver, suggesting that these genes may share some transcriptional regulatory components.

Through 5' external deletions, small internal deletions and insertions, and analysis of sequence elements in heterologous constructions, the transcriptionally active cis-acting regions in the apo B 5' flanking region were delineated and characterized in HepG2 and HeLa cells (summarized in Figure 7).

The most distal cis-acting region is located between -261 and -128 and has a negative effect on transcription in both HepG2 and HeLa cells. Deletion of this region increases transcription from the apo B promoter by about 3- to 4-fold in HepG2 cells and by at least the equivalent amount in HeLa cells (Figure 1B). It is likely that this region is composed of at least two individual elements, on from -261 to -152 and another from -138 to -128. This is suggested by the finding that deletion of sequences from -152 to -138 has very little effect on transcription (Figure 1B). It is important to note, however, that the level of transcription of the -128 deletion construction is still much lower in HeLa than in HepG2 cells. This observation, together with the fact that deletion of the region between -261 and -128 has the same relative effect in each cell type, suggests that this region does not contribute strongly to the cell-type specificity of apo B gene expression.

Cell-type specific expression does appear to be determined by two cis-acting element located between -128 and -70. The more distal of these two elements (-128 to -86) has a 4-fold positive effect in HepG2 cells and a 3- to 4-fold negative effect in HeLa cells (Figure 1B). One possible explanation for these results is that the region from -128 to -86 contains two distinct elements: one that is positive only in HepG2 cells and another that is negative only in HeLa cells. A second possibility is that the region contains a single element that has positive activity in HepG2 and negative activity in HeLa cells. The other cell-type specific element (-86 to -70) has a 13-fold positive effect in HepG2 cells but only a 2-fold positive effect in HeLa cells (Figure 1B). The crucial role of this element in determining hepatic-specific apo B expression is clearly seen when the internal deletion mutant -152B, missing this element, is compared to the wild type, template -152 (Figure 2). In HepG2 cells, this mutation results in a 70-fold decrease in apo B expression, whereas the HeLa cells, this deletion had very little effect. Thus, the -86 to -70 element, which is absolutely required for expression in HepG2 cells and only weakly positive in HeLa cells, contributes strongly to maintaining the cell-type specific pattern of apo B expression. The -128/-86 element and the -86/-70 element act together to cause a vary large differential effect in HepG2 versus HeLa cells and together account for the cell-type specific nature of apo B gene transcription.

Cis-acting DNA sequence elements that regulate gene transcription function as binding sites for the trans-acting protein factors which act as inhibitors in the method of the present invention. Using the gel mobility shift and DNAse I footprint assays, it can be demonstrated that the -86 to -70 element binds to proteins from extracts of hepatic nuclei. Designated B1 and B2 in Figures 4 and 6, these proteins probably represent the trans-acting factors responsible for the strong positive effect of the -86 to -70 element in hepatic cells. The appearance of two bands on the mobility shift gels that show the same sequence requirements could be due to either two distinct proteins that require the same sequence for binding or a single protein that occurs in two different forms, both of which bind to this sequence.

A second group of proteins, designated B3-B5 in Figures 4 and 6 were found to bind to the DNA sequence located between -70 an -62. Three bands appeared on mobility shift gels when HepG2 nuclear extracts were used as a source of protein, while only two appeared when mouse liver extracts were used. As with the relationship between B1 and B2, these bands may represent distinct proteins or different forms of the same protein. DNAse I footprinting analysis using both HepG2 and liver nuclear extracts showed a single protected region extending from -83 to -62 (Figures 5 and 6). This protected region probably represents the continuous binding sites of the B1/B2 protein(s) and the B3/B4/B5 protein(s).

It is interesting that no protein binding was observed to either the -261 to -128 negative region or to the positive -128 to -116 region. The most likely explanation is that both the HepG2 ad mouse liver extracts are missing the proteins that interact with these sequences in vivo. It is also possible that these proteins are present in the extracts but bind too weakly to be seen by either the gel mobility shift assay or the footprinting assay. This may also be the explanation for the lack of binding observed with HeLa cell nuclear extract.

In addition to the general transcriptional signals of the CAAT and TATA sequence elements (see Figure 7), the apo B regulatory region contains similarities to other apolipoprotein genes and to some other genes expressed in the liver. The apo B sequences from -92 to -85 contain an octamer sequence identical to the apo CIII gene sequence form -111 to -104 (see below, Table 1).

TABLE I

| Similarity of apo B 5′ flanking sequence elements with sequences from the regulatory regions of other human genes. | | | |
|---|---|---|---|
| Sequence | Gene/Location | Homology | Reference |
| Apo B positive region | (-116 to -86) | | |
| TCAGGCCC | apo B (-92 to -85) | | |
| TCAGGCCC | apo CIII (-111 to -104) | 8/8 | Reue, J. Biol. Chem. (in press) |
| Apo B positive region | (-86 to -70) | | |
| AGGCGCCCTTT | apo B (-81 to -71) | | |
| AGGtGaCCTTT | apo CIII (-83 to -75) | 9/11 | Reue, supra |
| gGGtGaCCTT | α1-antitrypsin (-84 to -76) | 7/9 | Simone, et al. EMBO J, 6, 2759-2766 (1987) |
| AGctGaCCTT | apo AI (-134 to -125) | 7/10 | Karaathanasis, et al. Nature, 304, 371-373 (1983) |
| AcGtGaCCTT | apo CII (-155 to -146) | 7/10 | Das, et al., J. Biol. Chem., 262, 4748-4793 (1987) |

In the case of the apo B gene, these could be the DNA bases governing positive expression of HepG2 and negative expression in HeLa cells. A similar behavior of the comparable region was shown for the apo CIII gene in HepG2 cells. In addition, sequences similar to the strongly positive apo B gene liver element located between -86 and -70 are found in the promoter regions of three other apolipoprotein genes and also in the promoter of the α1-antitrypsin gene (see Table 1). In the case of the apo CIII and the α1-antitrypsin genes, the sequence elements similar to the apo B positive region (-86 to -70) have been shown to be involved in hepatic specific transcription by Reue et al., J. Biol. Chem., 263, 6857-6864, 1988; Simone et al., EMBO J, 6, 2759-2766 (1987). The significance of these motifs in the other genes is unknown. The -86 to -70 element binds to at least one protein found in hepatic cell nuclear extracts. It has also been reported that the apo CIII and α1-antitrypsin sequence motifs that are similar to the -86 to -70 apo B sequence bind to hepatic proteins (Reue, supra, and Simone, supra). In the case of apo B and apo CIII, these proteins have been directly compared and appear to be the same protein. These similarities imply that at least some apolipoprotein genes share regulatory elements with eachother and with other liver expressed genes.

It should be noted that, although both of the sequence motifs governing tissue specific expression of the apo B gene are found in several liver specific genes, they are apparently not generally required for transcription in hepatic cells. Many liver specific genes do not appear to contain them, including albumin, α1-fetoprotein, transthyretin, apo E and apo A-II.

The experiments which follow were conducted to better understand the regulation of apo B gene expression. Cis-acting DNA elements regulating liver specific expression were defined by transient expression assays in human liver and epithelial cell lines, HepG2 and HeLa, respectively. In addition, transacting nuclear factors that interact with these regions were identified by gel mobility shift and DNAse I footprinting techniques.

## EXAMPLE 1

## Isolation of the Apo B Genomic Clone

A synthetic oligonucleotide 60 bases long corresponding to amino acids 1 to 20 of the mature apo B protein (23-26) is synthesized on an Applied Biosystems DNA synthesizer. The oligonucleotide is labeled at the 5' end with $^{32}$P by polynucleotide kinase and used to screen a human genomic library in lambda phage charon 4A [Lawn et al., Cell, 15, 1157-1174 (1978)]. Two overlapping lambda clones are isolated. One of these ( B) contains a fragment of the apo B gene extending from 8 kb upstream of the mRNA capsite to a site in the 2nd exon 7 kb downstream from the capsite.

The vector pKT (see Figure 1) is constructed by inserting the bacterial chloramphenicol actyltransferase (CAT) gene into pUC18 between the Hind III and Aat II sites, adjacent to the intact polylinker. The promoterless CAT gene insert is isolated from the plasmid pSVOCAT [Gorman et al., Mol. Cell. Biol., 2, 1044-1053 (1982)], as a Hind III-Bam HI fragment containing the complete CAT coding sequence followed by SV40 split and polyadenylation sequences. The plasmic pKTSV is produced by insertion of a 366 nucleotide Kpn I-Hind III fragment from pAW2 [Wildeman et all, EMBO J., 3, 3129-3133 (1984)] into the Kpn I and Hind III sites in the polylinker of pKT (see Figure 1).

The apo B-CAT construction (pKTB) is a 1 kb Pvu II fragment isolated from the lambda clone containing sequences from -900 to +122 with respect to the apo B mRNA capsite inserted into the Sma I site in the pKT polylinker. the site at +122 is in the 1st exon of the apo B gene and represents the 3' end of the apo B gene sequences in this and all subsequent constructions. Constructions -670, -261, -86 and -84 (see Figure 1B) are made by ligating the blunt ended Stu I - Pvu II (-670 to +122), Nco I-PVu II (-262 to +122) Ava I - Pvu II (-86 to +122) and Sma I - Pvu II (-84 to +122) fragments into the Sma I site of pKT. Deletion mutants with 5' endpoints at -173, -152, -138, -128, -116, -73 and -70 are made by Bal 31 nuclease digestion of the -261 construction linearized at the Sac I site of the polylinker. After Bal 31 nuclease digestion, the plasmid is blunt ended with T4 DNA polymerase and deoxyribonucleotide triphosphates. The inserts of variable lengths are. generated by digestion with Hind III, purified and ligated into the Sma I and Hind III sites of the pT vector. The 5' and 3' endpoints of each construction are determined by dideoxy sequencing.

The plasmic pCT (gift of J. Smith) contains a portion of the adenovirus 2 major late promoter (-50 to +33 relative to the start site of major-late transcription) and 58 bp of SV40 untranslated leader sequence (SV40 map units 5227 to 51712) located immediately upstream of the CAT gene in pKT (see Figure 3 and Smith et al., J. Biol. chem., in press for construction). Apo B sequences are inserted into the pCT polylinker immediately upstream of the adenovirus 2 major late promoter.

EXAMPLE 2

Cell Culture and Transfection Experiments

HepG2 and HeLa cells are maintained in DMEM supplemented with 10% or 5% fetal calf serum, respectively. For DNA transfection experiments, cells are plated in 60 mm culture dishes at approximately 35% confluence and cultured for 24 hours prior to transfection. DNA is transfected into cells by the calcium phosphate co-precipitation method. The media is changed once at 16 hours post-transfection, and the cells are harvested at 48 hours post-transfection and disrupted by freeze-thawing. The protein concentration of the extracts are determined with the BioRad reagent. To normalize for equal efficiency of transfection, a reference plasmid containing the bacterial $\beta$-galactosidase gene under the control of the SV40 early promoter is co-transfected with the test plasmid. Both the CAT and $\beta$-galactosidase activities are measured using the same amount of protein. The CAT assay is performed by the method of Gorman et al., supra. CAT activity is quantitated by scintillation counting of spots cut from chromatograms. The percent of $[^{14}C]$ chloraphenicol substrate converted to acetylated product is determined. $\beta$-Galactosidase activity is determined as described by Miller, in Experiments in Molecular Genetics, Cold Spring Harbor Laboratory, Cold Spring Harbor, New York, 1972. The percent CAT substrate converted is divided by the $\beta$-galactosidase activity to normalize for transfection efficiency. Most transfection experiments are repeated a minimum of three separate times and for each construction at least two different plasmid preparations are tested.

EXAMPLE 3

Nuclear Extract Preparation and Electrophoretic Mobility Shift Assay

Nuclear extracts from HeLa and HepG2 cells are prepared according to the method of Dignam et al., Nucl. Acids Res., 11, 1475-1489 (1983). Nuclear extracts from fresh mouse liver are prepared by the method of Gorski et al., Cell, 47, 767-776 (1986). Nuclear protein-DNA binding reactions are carried out in a volume of 20 $\mu$l containing 60 mM KCl, 20 mM Hepes (pH 7.9), 4% ficoll and 4mM MgCl$_2$. Poly dl-dC (1 $\mu$g) is added as a nonspecific competitor. A typical reaction contains 10,000 to 20,000 cpm (approximately 0.5 ng) of 5' labeled DNA with 0.5 to 5 $\mu$g of protein extract as indicated. After addition of extract, samples are incubated at room temperature for 30 minutes and analyzed by electrophoresis on nondenaturing 4% polyacrylamide gels in 0.25 x TBE (2.2 mM tris-borate, 2.2 mM boric acid, 0.5 mM EDTA). For competition experiments, conditions are as above except that appropriate competitor DNA (as indicated) is added to the reaction mixture prior to addition of extract.

EXAMPLE 4

DNAse I Footprinting Assay

The coding strand of a DNA fragment containing sequences from -261 to +122 with respect to the start site of transcription if 5' end labeled with $^{32}P$ and used as a template for footprinting. Ten ng (approximately 25,000 cmp) of the fragment is incubated with 12-48 $\mu$g of nuclear protein for 15 minutes on ice in a 50 $\mu$l

reaction containing 20 mM Tris 7.9, 60 mM KCL, 4 mM MgCl₂, 5 mM EDTA, 0.5 mM DTT, 5% glycerol, 2% polyvinyl alcohol and 1.25 $\mu$g of poly dI-dC (reaction buffer). The mixture is then incubated at room temperature for 2 minutes. 50 $\mu$l of a solution containing 6 ng of DNAse I was added and allowed to digest for 60 seconds at room temperature. DNAse I is inactivated by the addition of 100 $\mu$l of stop buffer containing 0.6 M Na Acetate, 1% SDS, 20 mM EDTA and 100 $\mu$g/ml of glycogen. The DNA is purified by phenol extraction and ethanol precipitation of the DNA, which is analyzed on a 6% polyacrylamide, 50% urea DBA sequencing gel. A G + A ladder, generated by sequencing [Maxam et al., Methods Enzymol., 65, 499-560 (1980)] the same end-labeled fragment, is run in the same sequencing gel.

## EXAMPLE 5

### Identification of Cis-acting Regulatory Regions

To study apo B gene expression, a portion of the apo B gene from approximately -900 to + 122 relative to the start site of transcription is inserted into the plasmid pKT immediately upstream of the CAT gene coding sequence (pKTB, see Figure 1A). A series of deletion mutants extending from -900 toward the transcription initiation site are constructed (see Figure 1B). These constructions are introduced into HepG2 and HeLa cells by the calcium phosphate co-precipitation method and the transient expression of CAT activity determined. In each experiment, replicate petri disches of cells are transfected with pKT or with pKTSV (an SV40 early promoter-CAT construction) (see Figure 1A). pKTSV is expressed in both HepG2 and HeLa cells and pKT is not expressed in either cell type. In selected experiments, correct mRNA initiation from the pKT apo B gene constructions is demonstrated by primer extension of the RNA isolated from transfected cells.

### A. Apo B Distal Negative Region (-900 to -128)

The -900 apo B-CAT hybrid was expressed in HepG2 but not HeLa cells (see Figure 1B). As successive 5' deletions are made to -128, there is a progressive increase in HepG2 expression to about 6 times the level of the -900 construction. This occurs in stages, suggesting a series of negative elements located between -900 and -670, -261 and -152 and -138 and -128. In HeLa cells, the -128 construction showed definite but low level expression not seen with the -900 construction. This suggests negative elements operating on sequences between -900 and -128 HeLa cells as well as HepG2 cells. However, these do not sufficiently account for the different levels of expression of the apo B-CAT constructions in these two cell types.

### B. Apo B Proximal Positive Region (-128 to -70)

In HepG2 cells, progressive deletions form the -128 endpoint indicate that this region contains two positive elements, one located between -128 and -86 and the other between -86 and -70 (see Figure 1B). Deletion of sequences from -128 to -86 caused a 4-fold decrease in CAT activity. Additional deletion from -86 to -70 causes a further 13-fold decrease in activity. Together, these two elements account for a 50-fold positive effect on apo B transcription in HepG2 cells. In contrast, deletion of the region between -128 and -116 in HeLa cells had no effect on transcription, and removal of the region between -116 and -86 actually increased transcription 3- to 4-fold. Deletion of the region from -86 to -70 results in a 2-fold decrease of expression. These results indicate that the -128/-70 region behaves differently in HepG2 compared to HeLa cells. Sequences between -128 and -116 act positively in HepG2 cells but appear to have no effect in HeLa cells. More strikingly, the region between -116 and -86 is mildly positive in HepG2 but negative in HeLa cells. Finally, the region between -86 to -70 has a strong (13-fold) positive activity in HepG2 cells but only a mild (2-fold) positive activity in HeLa cells.

To further characterize the proximal positive region between -128 and -70, several additional constructions are made. The -116 to -86 and the -86 to -70 elements are separated by the insertion of an 8 bp linker at the Sma I cleavage site between nucleotides -85 and -84. This was done in the context of both the -261 and -152 constructions (-261A and 152A, respectively, in Figure 2). In HepG2 cells, both linker insertion constructions have diminished expression compared to wild type, whereas in HeLa cells expression increases. Thus, an insertion at position -85 disrupts a positive activity in HepG2 and a mild negative activity in HeLa cells. This indicates that the contiguous relationship of both elements is important in regulating apo B expression in both cell types. To further examine the role of the -86 to -70 element, two mutants templates are constructed containing internal deletions of sequences between -84 and -72 (-152B) and -85 and -63 (-152C) (see Figure 2). In HepG2 cells, both deletions caused the complete loss of expression with a 70-fold decrease compared to the wild type -152 construction. In HeLa cells, these deletions produce only a very minor effect. These mutants confirm the major role of the -86 to -70 element in regulating liver expression of the apo B gene. Since the deletion mutants cause a complete loss of transcriptional activity, it appears that the positive element between -128 and -86 cannot function alone, but contributes to a higher level of expression in HepG2 cells only when the -86 to -70 region is intact.

In the context of the apo B gene promoter, the -116 to -70 region appears to consist of two elements that act together but with a different effect in HepG2 and HeLa cells. To test the ability of these elements to function outside of their normal context, they are placed individually and in combination into a test plasmid containing an abbreviated adenovirus major late promoter (pCT, see Figure 3). A double stranded oligonucleotide corresponding to the apo B gene sequences from -116 to -70 is synthesized and placed at -50 relative to the start of major late transcription in the pCT plasmid. While this fragment dramatically stimulates transcription in HepG2 cells, it has a negative effect on HeLa cells (see Figure 3). When this fragment is divided and the two elements tested separately, it was observed that the proximal element (-84 to -70) had a strong positive effect on transcription in HepG2 and a mild positive effect on HeLa cells (3- to 4-fold), whereas the -116 to -85 element was positive only in HepG2 cells. Thus, these two elements have the same effect on a heterologous promoter as they do in the context of the apo B promoter. The results of the mutational analysis of the apo B promoter are summarized schematically in Figure 7.

## C. Trans-acting Factors Interact with the Apo B Region between -84 and -62

The gel mobility shift assay is used to detect proteins that bind to the apo B gene region proximal to -128. In these experiments a radio-labeled apo B DNA fragment extending from -128 to +122 is incubated with nuclear extracts from HeLa, mouse liver and HepG2 cells (see Figure 4A). Both mouse liver and HepG2 cell nuclear extracts contain specific binding proteins that were competed away by excess unlabeled fragment. In the mouse liver experiments there were four major bands, designated B1, B2, B3 and B4 appear to be similar in mouse liver and HepG2 cell nuclear extracts, but only the HepG2 extract contains B5. Although several protein factors from HeLa cell nuclear extracts bound to the radio-labeled apo B fragment, these interactions are nonspecific since the bound proteins could not be compete away by excess unlabeled fragment.

To further localize the binding sites of the hepatic protein factors, a series of unlabeled DNA fragments missing specific regions between -128 and +122 are used in competition experiments with the radio-labeled -128 to +122 fragment (see Figure 4B). All five proteins failed to bind in the presence of competitors containing sequences from -116 to +122 and -84 to +122, suggesting that these factors bind downstream of -84. A competitor fragment containing sequences from -70 to +122 competed for the binding of B3, B4 ad B5 but not for B1 and B2 (see Figure 4B). Therefore, B1 and B2 must bind upstream of -70. Competition is also performed with DNA fragments prepared from the internal deletion mutants -152B and -152C, which are missing base pairs -84 to -72 and -85 to -63, respectively. Proteins B3, B4 and B5 are competed by the -152B template but not by the -152C template. Together, these results demonstrate that B3, B4 and B5 bind to sequences located between -70 and -62, while proteins B1 and B2 bind to the region between -84 and -70 with respect to the apo B transcription start site.

These results are confirmed by electrophoretic mobility shift assay using as the radio-labeled probes DNA fragments missing certain of the regions between -128 and +122 (see Figure 4C). The radio-labeled fragment -84 to +122 bound to all five proteins, whereas the fragment -70 to +122 bound only B3, B4 and B5. When a radio-labeled fragment prepared from -152C (containing the -85 to -63 internal deletion) is used, none of the protein bands appear.

In vitro footprinting is also used to map the binding sites of the trans-acting factors. In these experiments, a 5' end-labeled apo B gene DNA fragment between -261 and +122 is used and DNAse I protection is observed in the presence of HepG2 cell nuclear extract. As shown in Figure 5, a very strong footprint was observed spanning nucleotides -83 to -62. This is compatible with the electrophoretic mobility shift assay which found that all five proteins (B1-B5) bind in this region.

This invention may be embodied in other forms or carried out in other ways without departing from the spirit or essential characteristics thereof. The present disclosure is therefore to be considered as in all respects illustrative and not restrictive, the scope of the invention being indicated by the appended claims, and all changes which come within the meaning and range of equivalency are intended to be embraced therein.

## Claims

1. Use of a substance for the preparation of a composition for controlling the production of plasma low density lipoproteins (LDL) in a mammal, said substance comprising an agent capable of interfering with the transcription of the apo B gene in the liver.

2. Use according to Claim 1, wherein said agent is capable of interfering with the interaction of trans-acting proteins and the cis-acting DNA regulatory regions between the -128 to -60 sequence elements of the apo B gene.

3. Use according to Claims 1 or 2, wherein said composition may be used to treat premature atherosclerosis, familial hypercholesterolemia and familial hyperlipoproteinemia.

4. An agent for controlling the production of plasma low density lipoproteins (LDL) in a mammal, comprising a substance capable of interfering with the transcription of the apo B gene in the liver.

5. The agent of Claim 4, wherein said agent is capable of interfering with the interaction of trans-acting proteins and the cis-acting DNA regulatory regions between the -128 to -60 sequence elements of the apo B gene.

6. A pharmaceutical composition for controlling the production of plasma low density lipoproteins in a mammal comprising the agent of Claims 3 or 4 in combination with a pharmaceutically acceptable carrier or diluent.

7. A method for assessing the onset of an increase in the level of plasma low density lipoproteins (LDL) in a mammal comprising measuring the extent and rate transcription of the apo B gene in the liver of said mammal.

8. The method of Claim 7 wherein the extent and rate of transcription of said apo B gene is measured by measuring the binding of trans-acting proteins to the cis-acting DNA regulatory region consisting of the -128 to -60 sequence elements of the apo B gene.

9. An assay method for identifying substances for use in lowering plasma low density lipoprotein (LDL) levels which comprises evaluating the effects of these substances on the binding of trans-acting proteins to the cis-acting DNA regulatory region consisting of the -128 to -60 sequence elements of the apo B gene.

10. A method according to Claim 9, wherein the evaluation is conducted by adding to the trans-acting proteins, the appropriate radiolabeled DNA fragment and the potentially interfering substance, and measuring the resultant amount of bound protein by electrophoresis.

11. A method according to Claim 10, wherein the electrophoresis is conducted on a polyacrylamide gel.

FIGURE 1a

FIGURE 1b

EP 0 347 915 A2

FIGURE 2

|  | HepG2 Relative Activity | | | HeLa Relativity Activity | | |
|---|---|---|---|---|---|---|
| -261 ——— +122 | 202 | | | 1 | | |
| -261A ——— | 108 | | | 4 | | |
| -152 ——— +8bp | 603 | | | 4 | | |
| -152A ——— | 190 | | | 7 | | |
| -152B ——— +8bp | 9 | | | 6 | | |
| -152C ——— | 10 | | | 5 | | |
| -84 ——— | 127 | | | 20 | | |

CM }CM-AC  CM }CM-AC

FIGURE 3

Ad2MLP/SV40 +1
CAT

|  | HepG2 Relative Activity | | | HeLa Relative Activity | | |
|---|---|---|---|---|---|---|
| pCT | 1 | | | 5 | | |
| pCTf -84 -70 | 7 | | | 1 | | |
| pCTf -84 -70 | 13 | | | 18 | | |
| pCTf -84 -85 | 5 | | | 2 | | |

CM }CM-AC  CM }CM-AC

## FIGURE 4c

Template  128    84    70  152C
         |  |   |  |   |  |   |
         1  2   3  4   5  6   7

B1 —
B2 —

B3 —
B4 —
B5 —

F —

HepG2

## FIGURE 4a

1 2 3 4   5 6 7 8   9 10 11 12

F —

— B1
— B2

— B3
— B4
— B5

HeLa    LIVER    HepG2

## FIGURE 4b

Competitor    128  116  84  70  152C 152B    128  116  84  70  152C 152B
           1 2  3 4  5 6  7 8  9 10 11 12 13 14  15 16  17 18  19 20 21 22 23 24 25 26 27 28

F —

= B1
= B2

= B3
= B4
= B5

LIVER                    HepG2

FIGURE 5

G+A    1  2  3  4

-50—

-58—
-61—                                    ⌐ -62
-68—
-79—                                    ⌐ -83

-101—

-115—

-130—

FIGURE 7

                                    83
                          81        84
                          82        85
                          ├──────┤──┤

Hep G2

                          -86      -62

                          -128

                    ‖  —      +      ++

                                              +1

                                    ▼

-261              -116      -86  -70

HeLa

FIGURE 6

```
-260      -250      -240      -230      -220      -210      -200      -190
 |         |         |         |         |         |         |         |
CCATGGCCCCACTGAGACACAGGAAGGGCCGCGCCAGAGCACTGAAGACGCTTGGGGAAGGGAACCCACCTGGGACCCAG


-180      -170      -160      -150      -140      -130      -120      -110
 |         |         |         |         |         |         |         |
CCCCTGGTGGCTGCGGCTGCATCCCAGGTGGGCCCCCTCCCCGAGGCTCTTCAAGGCTCAAAGAGAAGCCAGTGTAGAAA


-100      -90       -80       -70       -60       -50       -40       -30
 |         |         |         |         |         |         |         |
AGCAAACAGGTCAGGCCCGGGAGGCGCCCGTTTGGACCTTTTGCAATCCTGGCGCTCTTGCAGCCTGGGCTTCCTATAAAT


-20       -10       +1
 |         |         ▽
GGGGTGCGGGCGCCGGCCGCGCATT
```